# EUROPEAN PATENT SPECIFICATION

(11) **EP 3 307 894 B1**
(45) Date of publication and mention of the grant of the patent: **28.01.2026**
(21) Application number: 16808223.8
(22) Date of filing: 08.06.2016
(51) Int. Cl.: C12N 15/86, C12N 15/867, C12N 15/113, A61K 48/00

(54) **NON-INTEGRATING VIRAL DELIVERY SYSTEM AND METHODS OF USE THEREOF**
SYSTEM UND VERFAHREN ZUR NICHTINTEGRALEN VIRALEN AUSGABE UND VERFAHREN ZUR VERWENDUNG DAVON
SYSTÈMES D'ADMINISTRATION VIRAUX NON INTÉGRATIFS ET PROCÉDÉS D'UTILISATION ASSOCIÉS

(30) Priority: 10.06.2015 US 201562173784 P
(43) Date of publication of application: 18.04.2018
(73) Proprietor: American Gene Technologies International Inc., Rockville, MD 20850 (US)
(72) Inventor: PAUZA, Charles, David, Rockville, MD 20850 (US); LAHUSEN, Tyler, Rockville, MD 20850 (US)
(74) Representative: J A Kemp LLP
(86) International application number: PCT/US2016/036519
(87) International publication number: WO 2016/200997

(56) References cited:
- WO-A1-2006/048215
- WO-A1-2015/078999
- WO-A2-2004/053137
- WO-A2-2007/133674
- WO-A2-2011/071476
- US-A- 5 674 703
- US-A1- 2003 096 787
- US-A1- 2010 120 155
- GORZIGLIA M I ET AL: "ELIMINATION OF BOTH E1 AND E2A FROM ADENOVIRUS VECTORS FURTHER IMPROVES PROSPECTS FOR IN VIVO HUMAN GENE THERAPY", JOURNAL OF VIROLOGY, THE AMERICAN SOCIETY FOR MICROBIOLOGY, US, vol. 70, no. 6, June 1996 (1996-06-01), pages 4173 - 4178, XP002006453, ISSN: 0022-538X
- VARGAS J ET AL: "NOVEL INTEGRASE-DEFECTIVE LENTIVIRAL EPISOMAL VECTORS FOR GENE TRANSFER", HUMAN GENE THERAPY, LIEBERT, US, vol. 15, no. 4, April 2004 (2004-04-01), pages 361 - 372, XP001205920, ISSN: 1043-0342, DOI: 10.1089/104303404322959515
- WENDELBURG ET AL: "AN ENHANCED EBNA1 VARIANT WITH REDUCED IR3 DOMAIN FOR LONG-TERM EPISOMAL MAINTENANCE AND TRANSGENE EXPRESSION OF ORIP-BASED PLASMIDS IN HUMAN CELLS", GENE THERAPY, NATURE PUBLISHING GROUP, GB, vol. 5, 6 October 1998 (1998-10-06), pages 1389 - 1399, XP002931315, ISSN: 0969-7128, DOI: 10.1038/SJ.GT.3300736
- WESTERHOUT ET AL: "A conditionally replicating HIV-based vector that stably expresses an antiviral shRNA against HIV-1 replication", MOLECULAR THERAPY : THE JOURNAL OF THE AMERICAN SOCIETY OF GENE THERAPY, ACADEMIC PRESS ; NATURE PUBLISHING GROUP, US, vol. 14, no. 2, 11 May 2006 (2006-05-11), pages 268 - 275, XP005524738, ISSN: 1525-0016, DOI: 10.1016/J.YMTHE.2006.03.018
- VARGAS ET AL.: "Conditionally replicating lentiviral-hybrid episomal vectors for suicide gene therapy.", ANTIVIRAL RES, vol. 80, no. 3, December 2008 (2008-12-01), pages 288 - 294, XP025684743

## Description

### FIELD

The present invention relates generally to the field of viral vectors and systems for the delivery of genes and other therapeutic, diagnostic, or research uses.

### BACKGROUND

Viral vectors can be used to transduce genes into target cells owing to specific virus envelope-host cell receptor interactions and viral mechanisms for gene expression. As a result, viral vectors have been used as vehicles for the transfer of genes into many different cell types including whole embryos, fertilized eggs, isolated tissue samples, tissue targets *in situ* and cultured cell lines. The ability to introduce and express foreign genes in a cell is useful for the study of gene expression and the elucidation of cell lineages as well as providing the potential for therapeutic interventions such as gene therapy, somatic cell reprogramming of induced pluripotent stem cells, and various types of immunotherapy.

In view of the wide variety of potential genes available for gene therapy, an efficient means of delivering these genes is desired. Several viral systems including murine retrovirus, adenovirus, and parvovirus (adeno-associated virus) have been developed as therapeutic gene transfer vectors. Many factors must be considered when developing viral vectors, including stability and control of expression, genome packaging capacity, and construct-dependent vector stability. In addition, *in vivo* application of viral vectors is often limited by host immune responses against viral structural proteins and/or transduced gene products.

An approach for the production of recombinant polypeptides or gene regulatory molecules, including small RNA, is the use of stable expression systems. These systems are based upon chromosomal integration of a transduced retrovirus genome (or at least a portion thereof) into the genome of the host cell, short-duration plasmid transfection, or non-integrating viral vectors also with limited half-life. The sites of gene integration are generally random, and the number and ratio of genes integrating at any particular site are often unpredictable; likewise, non-integrating plasmids or viral vectors also generate nuclear DNA but these species usually lack sequences required for DNA replication and continuous maintenance. Thus, vectors that rely on chromosomal integration result in permanent maintenance of the recombinant gene that may exceed the therapeutic interval, and plasmid or other non-replicating DNA is poorly controlled and may decay before completing a desired therapeutic interval.

Alternatives to stable expression systems for gene expression are transient expression systems. The expression of the latter gene expression systems is based on non-integrated plasmids, and hence the expression is typically lost as the cell undergoes division or the plasmid vectors are destroyed by endogenous nucleases. Thus, transient gene expression systems typically do not lead to sufficient expression over time and would traditionally require repeated treatments, which are generally undesirable.

WO 2006/048215 describes an adenoviral amplicon and producer cells for the production of replication-defective adenoviral vectors comprising a replication origin, an inducible E2 gene and a gene of intrest, as well as methods of preparation and uses thereof.

Gorziglia M I. et al., (1996) JOURNAL OF VIROLOGY, 70, P 4173-4178 describes the deletion of both E1 and E2A from an adenoviral vector to avoid production of viral proteins.

WO 2004/053137 describes a non-integrating viral "delivery system", which comprises a replication origin derived from a Papovavirus, an inducible replication initiation factor gene and a gene of interest.

WO 2011/071476 describes a non-integrating lentiviral vector comprised of an initiation factor gene encoding EBNA-1 under the control of an inducible promoter and further comprising an origin of replication and a gene of interest.

US 2003/096787 describes a non-integrating adenoviral delivery system comprising a cell expressing the E2 gene under an inducible promoter and a construct comprising an origin of replication and a gene of interest.

### SUMMARY

The present invention provides a viral delivery system comprising:
(a) a viral carrier, wherein the viral carrier has a defective integrase gene, wherein the viral carrier is a lentivirus, and wherein the defective integrase gene comprises
(b) a heterologous viral episomal origin of replication;
(c) a sequence encoding at least one initiator protein specific for the heterologous viral episomal origin of replication, wherein expression of the sequence encoding the at least one initiator protein specific for the heterologous viral episomal origin of replication is under the control of a drug inducible promoter; and
(d) at least one gene, shRNA, siRNA, miRNA, or other gene-silencing RNA of interest.

In some embodiment, the heterologous viral episomal origin of replication is from a papillomavirus, such as a bovine papillomavirus or a human papillomavirus, and the at least one initiator protein specific for the heterologous viral episomal origin of replication is the papillomavirus E1 and/or E2. In certain embodiments both E1 and E2 are present in the systems described herein.

Alternatively, in some embodiments, the heterologous viral episomal origin of replication is from Epstein-Barr virus or any of the related mammalian herpesviruses, and the at least one initiator protein specific for the heterologous viral episomal origin of replication is EBNA-1 or a specific initiator protein found in each individual herpesvirus.

In another aspect, the disclosed invention is direct to a pharmaceutical composition comprising the disclosed viral delivery system and a pharmaceutically acceptable carrier. In another aspect, the viral delivery system of the invention is for use in a method of treating or preventing a disease, comprising identifying a subject in need of treatment or prevention of a disease and administering to the subject a therapeutically effective amount of a viral delivery system according to the invention.

In some embodiments, the disease can be an infectious disease, such as Ebola virus, Lassa Fever virus or any viral agents that can be protected by specific antibodies, and in those embodiments, the gene, shRNA, siRNA, miRNA, and/or other gene-silencing RNA of interest can encode an antibody specific for Ebola virus or Lassa Fever virus or any other specific virus target.

In some embodiments, the disease can be an infectious disease such as *Staphylococcus aureus* or *Escherichia coli* or other bacterial, fungal, or protozoan pathogens, wherein the gene, shRNA, siRNA, miRNA, and/or other gene-silencing RNA of interest encodes an antibody targeted to reduce pathogen replication or block the activity of an exogenous pathogen-produced toxin.

In some embodiments, the disease is a genetic disease or disorder. In some embodiments, the genetic disease is alcohol abuse, and the gene, shRNA, siRNA, miRNA, and/or other gene-silencing RNA of interest encodes brain- derived growth factor.

In another aspect, the viral delivery system of the invention is for use in a method of treating nerve damage, comprising identifying a subject with nerve damage and administering to the subject a therapeutically effective amount of a viral delivery system according to the invention. In some embodiments, the gene, shRNA, siRNA, miRNA, and/or other gene-silencing RNA of interest is nerve growth factor.

In yet another embodiment, the viral delivery system of the invention is for use in a method of enhancing wound healing, comprising identifying a subject with a wound and administering to the subject a therapeutically effective amount of a viral delivery system according to the invention.. The wound can be, for example, resulting from an accident, injury, or surgery. In some embodiments, the gene, shRNA, siRNA, miRNA, and/or other gene-silencing RNA of interest is platelet-derived growth factor or vascular endothelial growth factor.

In another embodiment, the viral delivery system of the invention is for use in a method of enhancing recovery from traumatic skin burns, comprising identifying a subject with traumatic burns and administering to the subject a therapeutically effective amount of a viral deliver system according to the invention. Skin trauma may result from chemical or physical (heat or ultraviolet light or radiation) injury. In some embodiments, the gene, shRNA, siRNA, miRNA, and/or other gene-silencing RNA of interest encodes epidermal or keratinocyte growth factors, or other promoters of epidermal cell growth and differentiation.

In another embodiment, the viral delivery system of the invention is for use in a method of treating cancer or neoplastic disease comprising identifying a subject with cancer or neoplastic disease and administering to the subject a therapeutically effective amount of a viral delivery system according to the invention, wherein the gene, shRNA, siRNA, miRNA, and/or other gene-silencing RNA of interest encodes an antibody targeted at tumor antigens, or polypeptides and/or regulatory RNA intended to modify tumor susceptibility to immunotherapy, surgery, radiation, or chemotherapy.

In another embodiment, the disclosed invention is a system for delivering proteins and/or nucleic acids that comprise a system for gene deletion, modification, or re-sequencing. Current technology for modifying chromosomal genes lacks appropriate safety factors to prevent off-target effects. The proposed invention will ensure that genes encoding the DNA modifying systems of the current invention are depleted after a required therapeutic interval.

In another embodiment, the viral delivery system of the invention is for use in a method of enhancing bone healing, comprising identifying a subject with a wound and administering to the subject a therapeutically effective amount of a viral delivery system according to the invention. The bone fracture can be, for example, resulting from an accident, injury, trauma or surgery and may be bone nonunion, or acute fracture or required spinal fusion. In some embodiments, the gene, shRNA, siRNA, miRNA, and/or other gene-silencing RNA of interest encodes bone morphogenetic proteins 1-4 or cyclooxygenase-2 or vascular endothelial growth factor.

### BRIEF DESCRIPTION OF THE DRAWINGS

**Figure 1** depicts an exemplary virus within a virus vector according to an embodiment of the invention.
**Figure 2** depicts an exemplary vector-in-vector (VIV) embodiment (Vector 1) also containing an E1 initiator protein.
**Figure 3** depicts transduction results in 293 T cells using Vector 1.
**Figure 4** depicts an exemplary vector-in-vector embodiment also containing both E1 and E2 initiator proteins.

### DETAILED DESCRIPTION

Disclosed herein are non-integrating, episomally replicating viral vectors (*e.g*., lentiviral vectors) and methods of using the same. Episomally replicating vectors like the present invention can contain viral components from viruses like Papovaviridae (*e.g*., bovine papillomavirus or BPV) or Herpesviridae (*e.g*., Epstein Barr Virus or EBV) or Hepadnaviridae (*e.g*., Hepatitis B Virus or HBV). Episomal replicating vectors derived from these viruses may contain a replication origin and at least one viral trans-acting factor, *e.g*., an initiator protein, such as E1 for BPV and EBNA-1 for EBV or HBV polymerase or terminus binding protein of Adenovirus. The process of episomal replication typically incorporates both host cell replication machinery and viral trans-acting factors.

By using heterogeneous viral origins of replication, novel vectors can be engineered with an "off" switch for expression of viral proteins required to recognize the origin of replication. Switching off DNA replication of the vector will prevent further expression of the transduced genes, and the viral vector will disappear over time such that the transduced genes will no longer be present in the host cell. The disclosed systems and methods provide numerous improvements over the prior art, including the ability to prevent any toxic effects from over-expression or prolonged expression of transduced genes. Eliminating the gene once DNA replication ceases, prevents unwanted gene expression or knockdown in the future. Likewise, combining the benefits of episomal replication into a heterogeneous viral system provides for a platform that can safely and efficiently transduce genes of interest into a variety of cell types.

### Papillomavirus

Papillomaviruses replicate primarily as episomes in mammalian cells. Action of the viral E1 protein, which functions as a DNA helicase, on the viral origin of replication (ori) drives the production of hundreds to thousands of copies per cells depending on differentiation status of infected epithelial cells. Recognizing this property, several laboratories have tried to develop papillomavirus-based gene delivery systems using what became known as "shuttle plasmids." With a bacterial origin of replication to allow production of DNA in *E. coli* and a papillomavirus ori to allow episomal replication in mammalian cells, a number of studies were performed to demonstrate safety and durability of gene expression. In most cases, the ori came from bovine papillomavirus.

Papillomaviruses are evolved to infect epidermal and epithelial cells. As infected cells differentiate from basal to luminal surfaces, papillomaviruses increase DNA replication and copy number becomes very high until a tremendous dose of virus is released at the lumenal surface. This makes papillomaviruses highly contagious as is apparent from human papillomavirus. The surge in copy number is due primarily to host factors. However, this feature of papillomavirus can be exploited to target transient gene therapy to epidermal and epithelial surfaces.

Thus, certain features of papillomavirus can be used for driving expression and replication of an episomal vector, as well as targeting expression of the vector to specific cell types.

### Epstein Barr Virus (EBV)

Epstein-Barr virus (EBV), also known as human herpesvirus 4, is a member of the herpes virus family. It is one of the most common human viruses, and most people become infected with EBV at some point in their lives.

EBV is a double stranded DNA virus that contains approximately 85 genes, and that is known to infect B cells and epithelial cells. EBV is capable of both lytic and latent replication, the latter of which results in a circularized version of the EBV genome translocating to the host cell nucleus where it may be replicated by host cell DNA polymerases.

EBV can undergo latent replication via at least three distinct pathways, but each one involves the expression of Epstein-Barr virus nuclear antigen 1 (EBNA-1), a protein that binds the episomal replication origin and mediates partitioning of the episome during division of the host cell. EBNA-1 plays an integral role in EBV gene regulation, replication, and episomal maintenance.

Due to its natural tropism and ability to replicate extrachromosomally, EBV is of interest for use as a viral vector. Such vectors will often be mutated to delete EBV's viral oncogenes and/or the essential lytic genes. However, EBV's utility as a viral vector has thus far been limited to *ex vivo* and research purposes.

### Hepatitis B virus (HBV)

Hepatitis B virus (HBV) is a member of the hepadna virus family. It is a common human virus associated with progressive liver fibrosis, hepatitis and hepatocellular carcinoma.

HBV is a double stranded DNA virus that replicates through an RNA intermediate and depends on a viral polymerase. Stable maintenance of HBV in liver cells is due to the presence of covalently-closed viral DNA circular forms that are difficult to eradicate.

Due to its natural tropism and ability to replicate extrachromosomally, HBV as a vector provides significant potential advantages for targeting liver cells and delivering therapeutic genes. The unique life cycle for HBV provides a unique method for regulating episomal DNA levels through controlled expression of the viral polymerase required to convert an RNA intermediate into viral DNA.

### Retrovirus

Retrovirus is a virus family characterized by encoding a reverse transcriptase capable of generating DNA copies from RNA templates and integration of proviruses into the host cell chromosome. Lentivirus is a genus of retroviruses that can deliver a significant amount of viral nucleic acid into a host cell. Lentiviruses are characterized as having a unique ability to infect/transduce non- dividing cells, and following transduction, lentiviruses integrate their nucleic acid into the host cell's chromosomes.

Infectious lentiviruses have three main genes coding for the virulence proteins *gag, pol,* and *env,* and two regulatory genes including *tat* and *rev.* Depending on the specific serotype and virus, there may be additional accessory genes that code for proteins involved in regulation, synthesis, and/or processing viral nucleic acids and other replicative functions.

Moreover, lentiviruses contain long terminal repeat (LTR) regions, which may be approximately 600 nt long. LTRs may be segmented into U3, R, and U5 regions. LTRs can mediate integration of retroviral DNA into the host chromosome via the action of integrase. Alternatively, without functioning integrase, the LTRs may be used to circularize the viral nucleic acid.

Viral proteins involved in early stages of lentivirus replication include reverse transcriptase and integrase. Reverse transcriptase is the virally encoded, RNA-dependent DNA polymerase. The enzyme uses a viral RNA genome as a template for the synthesis of a complementary DNA copy. Reverse transcriptase also has RNaseH activity for destruction of the RNA-template. Integrase binds both the viral cDNA generated by reverse transcriptase and the host DNA. Integrase processes the LTR before inserting the viral genome into the host DNA. *Tat* acts as a trans-activator during transcription to enhance the initiation and elongation of RNA copies made from viral DNA. The *rev* responsive element acts post-transcriptionally, regulating mRNA splicing and transport to the cytoplasm.

### Vector-in-Vector Technology

Disclosed herein is a novel vector-in-vector (VIV) system that can precisely regulate the delivery and expression of genes by combining desirable features from various viral species. Many viral vectors, including lentivirus (LV) platforms, are known in the art. LV benefits from ease of manufacturing and flexible targeting characteristics, but lentiviral transduction, like most other forms of stable transduction, results in chromosomal integration of the LV payload. The property of chromosomal integration can be abolished through mutations that inactivate the viral integrase gene.

Avoiding chromosomal integration reduces the barrier to *in vivo* gene delivery. Even integration-defective LV can have a background frequency of integration, and any DNA molecule can find rare homologies to recombine with host sequences; but these rates of integration are exceptionally rare and generally not clinically significant.

As examples, the papillomavirus ori plus E1 protein, or the EBV ori plus EBNA-1 or the Hepadnavirus termini plus viral polymerase can be added as part of the genetic cargo of a heterologous virus that would not ordinarily be able to be maintained episomally. Incorporating this heterogeneous viral replication machinery into a lentiviral vector leaves approximately 5 kb of additional cargo space available to accommodate therapeutic genes of interest.

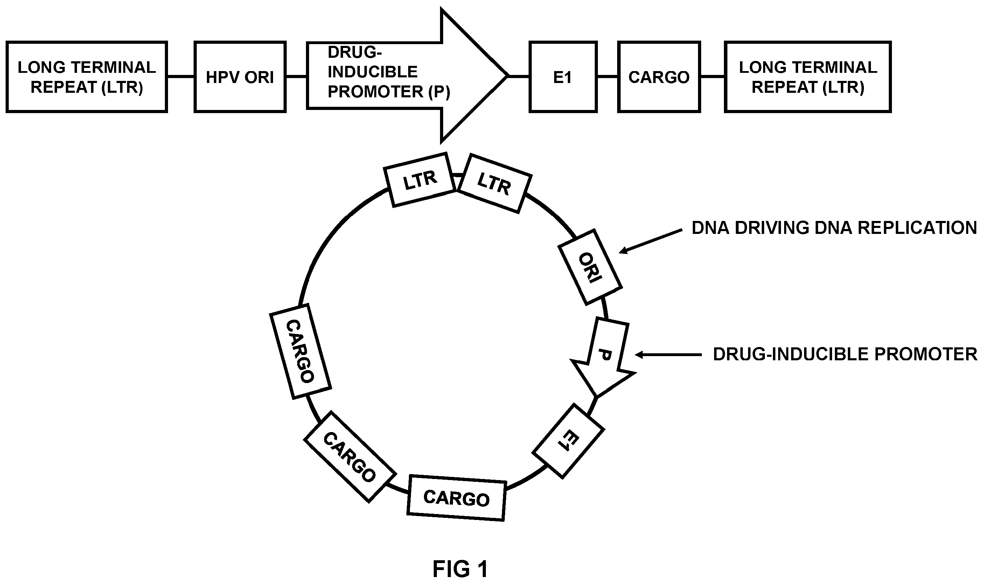

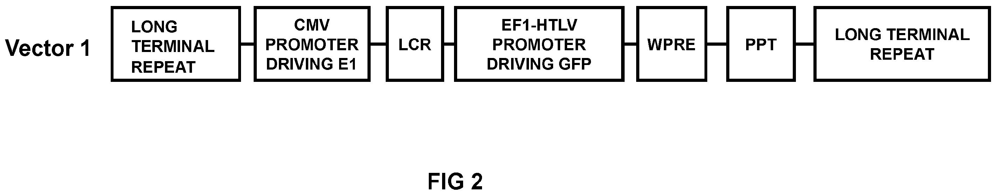

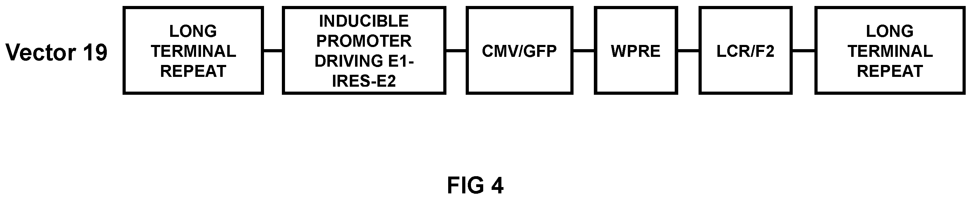

Additionally, other control elements can be incorporated into the disclosed VIV system. For instance, the expression of E1 or EBNA-1 or HBV polymerase can be driven by an inducible promoter. Numerous types of inducible promoters are known in the art, and for the purposes of this invention, inducible promoters include promoters that respond to antibiotics (*i.e.*, tetracyclines, aminoglycosides, penicillins, cephalosporins, polymyxins, *etc.*) or other drugs. For example, a method of using the disclosed viral system could employ tetracycline-inducible gene expression that depends upon a constant supply of the drug for expression of the cargo genes. A compound used to induce the inducible promoter may be added once or repeatedly depending on the duration of episomal replication and timing of cargo delivery that is desired. DNA replication and maintenance of the episome depends on E1 and possibly E2 or EBNA-1 induction, which in turn depends upon an inducer of gene expression (*i.e.*, tetracycline). An exemplary diagram of a VIV system appears in Figure 1. Another exemplary diagram of a VIV system appears in Figure 2. As shown in Figure 2, an E1 initiator protein is present and the cargo is GFP under an EF1-HTLV promoter. Another exemplary diagram of a VIV system appears in Figure 4 where the genetic cargo is represented by a CMV/GFP expression cassette. Cargo gene sequences can be amplified by polymerase chain reaction (PCR) using synthetic oligonucleotide primers identical to the 5' end of the cargo gene and complementary to the 3' end of the cargo gene. The 5' primer can be extended from its 5' end with a recognition site for an endonuclease. The 3' primer can also be extended at its 3' end with the complement for an endonuclease recognition. The resulting amplified cargo gene sequences can be annealed into a suitable vector, such as a lentiviral vector. Non-limiting examples of the genetic cargo include CMV/VEGF, CMV/anti-epidermal growth factor receptor (EGFR), or miRNA suppressing C-C chemokine receptor type 5 (CCR5).

Suitable expression of the cargo may be determined by an appropriate assay. For example, DNA copy numbers can be measured by quantitative PCR. Protein products translated from any of Vector 1 or Vector 19 (described herein) can be measured, for example, by analytical flow cytometry. An ELISA assay may be used to detect the presence of certain cargo, such as a secreted protein, such as VEGF. A Western blot technique may also be used to detect certain cargo such as an antibody, such as anti-EGFR. Further, monitoring a reduction in cell surface expression of a cargo protein, such as a chemokine receptor such as CCR5, can also be employed.

As shown in Figure 1, the disclosed VIV can comprise at least one gene or sequence of interest. The genes or sequences incorporated into the VIV will depend upon the purpose of the VIV. Referring to Figure 1, lentivirus is packaged with an integrase-defective system or transduction is performed in the presence of clinical drugs used to block integrase activity (*e.g.*, Dolutegravir or Raltegravir). Failing to integrate, the linear double strand vector DNA will generally circularize using host enzymatic machinery. A drug inducible promoter can be activated to express E1 and/or E2 protein if desired, which will in turn drive DNA replication. Therapeutic cargo will be expressed from cassettes. In various embodiments, the compound that induces the inducible promoter (also referred to herein as an "inducer") is withdrawn or terminated. Termination of the inducer will down regulate E1 and/or E2 synthesis. In further embodiments, E1 and/or E2 production is effectively terminated. In either event, this will lead to declining levels of episomal DNA and eventually elimination of the vector construct. Figures 1 and 2 depict a VIV system containing E1. Figure 4 depicts a VIV system containing both E1 and E2 on a single viral vector. In a preferred embodiment, in order to express both E1 and E2 from the same mRNA, an internal ribosome entry site (IRES) is added to allow for re-initiation of protein translation.

For instance, the gene encoding platelet-derived growth factor (PDGF) could be incorporated as a gene along with shRNA, siRNA, miRNA, and/or other gene-silencing RNA of interest into a VIV used to promote wound healing. The disclosed VIV system is not limited to a type of gene or sequence that can be expressed. Thus, the disclosed VIV can incorporate numerous therapeutic or prophylactic genes or sequences, for instance, sequences that encode antibodies directed to an antigen associated with an infectious disease or cancer (including antigens on replicating pathogens and antigens that are exogenous toxins and antigens on tumor cells), platelet derived growth factor, vascular endothelial growth factor, brain derived growth factor, nerve growth factor, human growth factor, human chorionic gonadotropin, cystic fibrosis transmembrane conductance regulator (CFTR), dystrophin or dystrophin-associated complex, phenylalanine hydroxylase, lipoprotein lipases, α- and/or β-thalassemias, factor VIII, bone morphogenetic proteins 1-4, cyclooxygenase 2, vascular endothelial growth factor, chemokine receptor CCR5, chemokine receptor CXCR4, chemokine receptor CXCR5, antisense DNA or RNA against autoimmune antigens involved in colitis, inflammatory bowel disease or Crohn's disease, small interfering RNA that are involved in addiction including miRNA regulating neural attenuation to opiates or alcohol, tumor suppressor genes, genes regulating cell survival including pro- or anti-apoptosis genes and pro- or anti-autophagy genes, genes encoding radiation resistance factors, genes encoding light emitting proteins used for tracking tumor cell metastasis or other cell trafficking phenomena, or a variety of other therapeutically useful sequences that may be used to condition the body for maximum effect of radiation, surgical or chemotherapeutics or to protect tissues against radiation, surgical or chemotherapeutics, to modify the host or graft tissues to improve organ transplantation or to suppress hyprerreactivity especially in the airway.

Maintaining genes in episomal form in a VIV system builds in a safety switch. If the gene product is toxic, withdrawal of the inducer molecule will terminate DNA replication, episome numbers will subsequently decline, and the gene and vector will disappear. Unlike traditional regulated gene expression as the safety switch, the disclosed expression construct is degraded by endogenous nucleases and diluted by cell division until it has effectively disappeared, thereby preventing any short- or long-term breakthrough expression.

Maintaining a gene, shRNA, siRNA, miRNA, and/or other gene-silencing RNA of interest in episomal form also allows for regulating the copy number over a broad range and at much higher levels than is achieved by traditional lentivirus transduction.

The disclosed VIV system presents numerous benefits. For instance, episomal DNA is less susceptible to chromosomal modification, which can lead to gene silencing of traditional transduction vectors. Likewise, VIV episomal DNA vectors support active gene delivery at least over short- to medium-range time intervals of about 1 to about 4 months, and possibly longer. In other embodiments of the invention, episomal DNA vectors support active gene delivery over a period of about 1, about 2, about 3, about 4, about 5, about 6, about 7, about 8, about 9, about 10, about 11, or about 12 weeks. In other embodiments, episomal DNA vectors support active gene delivery over a period of about 1 month, 2 months, 3 months, 4 months, 5 months, 6 months, 7 months, 8 months, 9 months, 10 months, 11 months, 12 months, or longer. Any combination of these time periods can also be used, *e.g.,* 1 month and 1 week, or 3 months and 2 weeks.

### Methods

In some embodiments, the viral delivery system of the invention is for use in methods of administering a VIV vector to a patient in need thereof, wherein the VIV vector encodes at least one, at least two, at least three, at least four, or at least five genes of interest. Given the versatility and therapeutic potential and the disclosed VIV system, a VIV system of the invention may encode genes or nucleic acid sequences that include but are not limited to an antibody directed to an antigen associated with an infectious disease or a toxin produced by the infectious pathogen, platelet derived growth factor, vascular endothelial growth factor, brain derived growth factor, nerve growth factor, human growth factor, human chorionic gonadotropin, cystic fibrosis transmembrane conductance regulator (CFTR), dystrophin or dystrophin-associated complex, lipoprotein lipases, α- and/or β-thalassemias, factor VIII, bone morphogenetic proteins 1-4, cyclooxygenase 2, vascular endothelial growth factor, chemokine receptor CCR5, chemokine receptor CXCR4, chemokine receptor CXCR5, antisense DNA or RNA against autoimmune antigens involved in colitis, inflammatory bowel disease or Crohn's disease, small interfering RNA that are involved in addiction including miRNA regulating neural attenuation to opiates or alcohol, tumor suppressor genes, genes regulating cell survival including pro- or anti-apoptosis genes and pro- or anti-autophagy genes, genes encoding radiation resistance factors, genes encoding light emitting proteins used for tracking tumor cell metastasis or other cell trafficking phenomena, or a variety of other therapeutically useful sequences that may be used to condition the body for maximum effect of radiation, surgical or chemotherapeutics or to protect tissues against radiation, surgical or chemotherapeutics, to modify the host or graft tissues to improve organ transplantation or to suppress hyprerreactivity especially in the airway.

### Infectious Disease

The disclosed compositions and methods can provide protection against disease, particularly to individuals temporarily residing in a high-risk region. Several key targets for genetic therapy involve a great deal of risk if the gene product is chronically expressed. One exemplary application is the prophylactic delivery of monoclonal antibodies, including protective antibodies against a lethal viral agent, which are necessary to protect individuals moving through an endemic region (*e.g*., military and aid workers entering an Ebola-infected region). Vaccines are largely untested for diseases such as Ebola or Lassa Fever virus or Dengue fever or Chikungunya virus or Plasmodium spp. causing malaria, and chronic expression of prophylactic antibody genes through the use of integrating vectors carries unknown health risks. Thus, there is a significant medical need for effective antibody expression that must be high but transient. The disclosed VIV system and methods of delivering high copy numbers of a gene, shRNA, siRNA, miRNA, and/or other gene-silencing RNA of interest for a limited period satisfy this medical need.

In one embodiment, the viral delivery system of the invention is for use in methods of treating, preventing, or minimizing conditions, symptoms, or side effects associated with infectious disease. In some embodiments, the infectious disease can be human immunodeficiency virus (HIV), human T cell leukemia virus, Ebola virus, Lassa fever virus, dengue fever, Zika virus, malaria, tuberculosis, rabies, vaccinia virus or other infectious diseases. In some embodiments, a VIV vector can be administered prophylactically or following infection with an infectious disease.

### Wound Healing

In another embodiment, the viral delivery system of the invention is for use in methods of treating, preventing, or minimizing conditions, symptoms, or side effects associated with wound healing. The disclosed composition can be administered systemically or directly to a wound after an accident, injury, or surgery. In the case of surgery, a VIV vector may be administered prophylactically in order to expedite healing. In the case of a wound from an accident, injury, or surgery, a VIV vector may be administered sometime after the formation of the wound. For instance, the VIV vector may be administered within about 1, about 2, about 3, about 4, about 5, about 10, about 12, about 24, about 36, about 48, about 60, about 72, about 84, about 96, about 108, about 120, or about 168 hours of the formation of a wound.

Another application of the compositions of the invention is transient delivery of VIV constructs capable of expressing platelet growth factor that would accelerate wound healing. A high dose of platelet-derived growth factor (PDGF) is required very quickly but transiently. The disclosed system and methods are ideal for this type of application.

Additional short-term applications include expression of brain-derived growth factor for intermittent treatment of alcohol abuse, nerve growth factor for spinal cord regeneration, and topical applications for skin conditions.

### Bone Disease or Injury

In one embodiment, the viral delivery system of the invention is for use in a method of enhancing bone healing, comprising identifying a subject with a bone injury and administering to the subject a therapeutically effective amount of a viral delivery system according to the invention. The bone injury can be, for example, resulting from an accident, injury, or surgery and may be bone nonunion, or acute fracture or required spinal fusion. In some embodiments, the gene, shRNA, siRNA, miRNA, and/or other gene-silencing RNA of interest encodes bone morphogenetic proteins 1-4 or cyclooxygenase-2 or vascular endothelial growth factor.

In one embodiment, the viral delivery system of the invention is for use in a method of enhancing bone healing, comprising identifying a subject with a bone disease and administering to the subject a therapeutically effective amount of a viral delivery system according to the invention. The bone disease can be, for example, resulting from an accident, injury, or surgery and may be bone nonunion, or acute fracture or required spinal fusion. Additionally, the bone disease may be from low bone density, low blood flow to the bone, aging, hereditary conditions, and the like. In some embodiments, the gene, shRNA, siRNA, miRNA, and/or other gene-silencing RNA of interest encodes bone morphogenetic proteins 1-4 or cyclooxygenase-2 or vascular endothelial growth factor.

### Hereditary Genetic Disease

| **Table 1** | | |
|---|---|---|
| **Disorder** | **Mutation** | **Chromosome** |
| 22q11.2 deletion syndrome | D | 22q |
| Alpha-1-anti-trypsin disorder 14q32 Angelman syndrome | P | 14q32 |
| Canavan disease | | 17p |
| Charcot-Marie-Tooth disease | | |
| Color blindness | P | X |
| Cri du chat | D | 5 |
| Cystic fibrosis | P | 7q |
| Down syndrome | C | 21 |
| Duchenne muscular dystrophy | D | Xp |
| Haemochromatosis | P | 6 |
| Haemophilia | P | X |
| Klinefelter syndrome | C | X |
| Neurofibromatosis | | 17q/22q/? |
| Phenylketonuria | P | 12q |
| Polycystic kidney disease | P | 16 (PKD1) or 4 (PKD2) |
| Prader-Willi syndrome | DC | 15 |
| Sickle-cell disease | P | 11p |
| Tay-Sachs disease | P | 15 |
| Turner syndrome | C | X |

In another embodiment, the viral delivery system of the invention is for use in methods of treating, preventing, or minimizing conditions, symptoms, or side effects associated with a hereditary genetic disease. Several examples of such hereditary genetic diseases are given in Table 1, along with the causal type of mutation and chromosome involved using the nomenclature below:
P - Point mutation, or any insertion/deletion entirely inside one gene
D - Deletion of a gene or genes
C - Whole chromosome extra, missing, or both (see Chromosome abnormality)
T - Trinucleotide repeat disorders: gene is extended in length

Current gene therapy includes efforts to edit genomic DNA through gene deletion, replacement, or re-sequencing. Various gene therapy systems known in the art, including Talen, CRISPR-Cas9, zinc finger endonuclease and others, rely on delivery of genetic material by lentivirus transduction. But, unlike the disclosed invention, these systems may have unexpected consequences if left active in cells for extended periods because active chromosome modification systems may alter unexpected sites, leading to new genetic diseases including cancer. Truly practical systems for modification of host DNA require transient, well-regulated expression through methods such as the method disclosed herein.

Thus, in one embodiment, the viral delivery system of the invention is for use in a method of treating a hereditary genetic disease, comprising identifying a subject with a hereditary genetic disease and administering to the subject a therapeutically effective amount of a viral delivery system according to the invention. The hereditary genetic disease can be, for example, the diseases listed in Table 1, and in some embodiments, the gene, shRNA, siRNA, miRNA, and/or other gene-silencing RNA of interest encodes non-mutated versions of the genes listed in Table 1.

It is also possible to incorporate a guide RNA target sequence into the disclosed VIV vectors. Guide RNA are sequences used to target gene editing machinery to specific sites within the host genome that are mutated or otherwise require correction. Inclusion of guide RNA within the cargo of a VIV vector allows for a modification of a section of a chromosome that requires correction, and the same modification will occur within VIV to accelerate degradation and/or dilution by the host. Thus, in certain embodiments of the invention, the disclosed viral delivery system comprises at least one guide RNA.

### Ex vivo modification of cells or tissues

In another embodiment, VIV vectors may be used to modify cells or tissues that are used for disease therapy. Cells may include, without limitation, primary cells such as lymphocytes, stem cells, epithelial cells, neural cells and others. For example, VIV vectors may be used to modify lymphocytes that are redirected to specific disease including cancer, infectious disease or autoimmunity, and where long-term presence of genetically modified cells poses a health risk. For example, VIV vectors may also be used to program pluripotent stem cells that require high levels of transcript factors for a defined interval and where the long-term presence of an integrated viral vector is undesirable. Epithelial cells that may be used for synthetic skin or other applications may require the expression of trophic or growth factors during the initial treatment that would be deleterious to function of the normal tissue after treatment and are best delivered by VIV vectors.

### Doses and Dosage Forms

The disclosed VIV vectors allow for short, medium, or long-term expression of genes or sequences of interest and episomal maintenance of the disclosed vectors. Accordingly, dosing regimens may vary based upon the condition being treated and the method of administration.

In one embodiment, VIVs may be administered to a subject in need in varying doses. Specifically, a subject may be administered ≥ 10⁶ infectious doses (where 1 dose is needed on average to transduce 1 target cell). More specifically, a subject may be administered ≥ 10⁷, ≥ 10⁸, ≥ 10⁹, or ≥ 10¹⁰ infectious doses. Upper limits of VIV dosing will be determined for each disease indication and will depend on toxicity/safety profiles for each individual product or product lot.

Additionally, a VIV of the present invention may be administered once or twice a day. Alternatively, a VIV may be administered to a subject in need once a week, once every other week, once every three weeks, once a month, every other month, every three months, every six months, every nine months, once a year, every eighteen months, every two years, every 36 months, or every three years.

In one embodiment, VIVs are administered as a pharmaceutical composition. In one embodiment, the pharmaceutical composition comprising VIV can be formulated in a wide variety of nasal, pulmonary, oral, topical, or parenteral dosage forms for clinical application. Each of the dosage forms can contain various disintegrating agents, surfactants, fillers, thickeners, binders, diluents such as wetting agents or other pharmaceutically acceptable excipients. The pharmaceutical composition comprising a VIV can also be formulated for injection.

The VIV composition can be administered using any pharmaceutically acceptable method, such as intranasal, buccal, sublingual, oral, rectal, ocular, parenteral (intravenously, intradermally, intramuscularly, subcutaneously, intracisternally, intraperitoneally), pulmonary, intravaginal, locally administered, topically administered, topically administered after scarification, mucosally administered, via an aerosol, or via a buccal or nasal spray formulation.

Further, the VIV composition can be formulated into any pharmaceutically acceptable dosage form, such as a solid dosage form, tablet, pill, lozenge, capsule, liquid dispersion, gel, aerosol, pulmonary aerosol, nasal aerosol, ointment, cream, semi-solid dosage form, and a suspension. Further, the composition may be a controlled release formulation, sustained release formulation, immediate release formulation, or any combination thereof. Further, the composition may be a transdermal delivery system.

In another embodiment, the pharmaceutical composition comprising a VIV can be formulated in a solid dosage form for oral administration, and the solid dosage form can be powders, granules, capsules, tablets or pills. In yet another embodiment, the solid dosage form can include one or more excipients such as calcium carbonate, starch, sucrose, lactose, microcrystalline cellulose or gelatin. In addition, the solid dosage form can include, in addition to the excipients, a lubricant such as talc or magnesium stearate. In some embodiments, the oral dosage form can be immediate release, or a modified release form. Modified release dosage forms include controlled or extended release, enteric release, and the like. The excipients used in the modified release dosage forms are commonly known to a person of ordinary skill in the art.

In a further embodiment, the pharmaceutical composition comprising a VIV can be formulated as a sublingual or buccal dosage form. Such dosage forms comprise sublingual tablets or solution compositions that are administered under the tongue and buccal tablets that are placed between the cheek and gum.

In yet a further embodiment, the pharmaceutical composition comprising a VIV can be formulated as a nasal dosage form. Such dosage forms of the present invention comprise solution, suspension, and gel compositions for nasal delivery.

In one embodiment, the pharmaceutical composition can be formulated in a liquid dosage form for oral administration, such as suspensions, emulsions or syrups. In other embodiments, the liquid dosage form can include, in addition to commonly used simple diluents such as water and liquid paraffin, various excipients such as humectants, sweeteners, aromatics or preservatives. In particular embodiments, the composition comprising VIV or a pharmaceutically acceptable salt thereof can be formulated to be suitable for administration to a pediatric patient.

In one embodiment, the pharmaceutical composition can be formulated in a dosage form for parenteral administration, such as sterile aqueous solutions, suspensions, emulsions, non-aqueous solutions or suppositories. In other embodiments, the non-aqueous solutions or suspensions can include propyleneglycol, polyethyleneglycol, vegetable oils such as olive oil or injectable esters such as ethyl oleate. As a base for suppositories, witepsol, macrogol, tween 61, cacao oil, laurin oil or glycerinated gelatin can be used.

The dosage of the pharmaceutical composition can vary depending on the patient's weight, age, gender, administration time and mode, excretion rate, and the severity of disease.

### Definitions

Words that are not specifically defined herein will be understood to have a meaning consistent with that as understood by persons of ordinary skill in the art.

As used herein, the term "about" will be understood by persons of ordinary skill in the art and will vary to some extent depending upon the context in which it is used. If there are uses of the term which are not clear to persons of ordinary skill in the art given the context in which it is used, "about" will mean up to plus or minus 10% of the particular term.

A "treatment" is intended to target the disease state and combat it, *i.e.,* ameliorate or prevent the disease state. The particular treatment thus will depend on the disease state to be targeted and the current or future state of medicinal therapies and therapeutic approaches. A treatment may have associated toxicities.

The terms "administration of" or "administering" an active agent should be understood to mean providing an active agent of the invention to the subject in need of treatment in a form that can be introduced into that individual's body in a therapeutically useful form and therapeutically effective amount.

The term "therapeutically effective amount" refers to a sufficient quantity of the active agents of the present invention, in a suitable composition, and in a suitable dosage form to treat or prevent the symptoms, progression, or onset of the complications seen in patients suffering from a given ailment, injury, disease, or condition. The therapeutically effective amount will vary depending on the state of the patient's condition or its severity, and the age, weight, *etc.*, of the subject to be treated. A therapeutically effective amount can vary, depending on any of a number of factors, including, *e.g*., the route of administration, the condition of the subject, as well as other factors understood by those in the art.

The term "treatment" or "treating" generally refers to an intervention in an attempt to alter the natural course of the subject being treated, and can be performed either for prophylaxis or during the course of clinical pathology. Desirable effects include, but are not limited to, preventing occurrence or recurrence of disease, alleviating symptoms, suppressing, diminishing or inhibiting any direct or indirect pathological consequences of the disease, ameliorating or palliating the disease state, and causing remission or improved prognosis.

The terms "individual," "host," "subject," and "patient" are used interchangeably herein.

As used herein, "expression," "expressed," or "encodes" refers to the process by which polynucleotides are transcribed into mRNA and/or the process by which the transcribed mRNA is subsequently being translated into peptides, polypeptides, or proteins. Expression may include splicing of the mRNA in a eukaryotic cell or other forms of post-transcriptional modification or post-translational modification.

The following examples are given to illustrate the present invention. It should be understood, however, that the invention is not to be limited to the specific conditions or details described in these examples.

### Examples

### Example 1 - Use of VIV in the Treatment of Infectious Disease

This example demonstrates the use of a disclosed VIV, as demonstrated by a non-limiting example exemplified in Figure 1, in the treatment of Ebola virus or other infectious diseases. In this example, at least one of the "cargo" regions shown in Figure 1 encodes an antibody that specifically targets Ebola virus.

Subjects suspected of having or diagnosed as having Ebola receive administrations of a therapeutically effective amount of a VIV encoding an antibody that specifically targets Ebola virus, either alone or in combination with one or more additional agents for the treatment or prevention of Ebola. VIV encoding an antibody that specifically targets Ebola virus and/or additional agents are administered orally, intranasally, intrathecally, intraocularly, intradermally, transmucosally, iontophoretically, topically, systemically, intravenously, subcutaneously, intraperitoneally, or intramuscularly according to methods known in the art or as described herein. Subjects will be evaluated daily for the presence and/or severity of signs and symptoms associated with Ebola, including, but not limited to, *e.g.,* fever, fatigue, malaise, weakness, reddened eyes, joint and muscle pain, headache, nausea, vomiting, hemorrhage, and death. Treatments are maintained until such a time as one or more signs or symptoms of Ebola are ameliorated or eliminated.

It is rationally predicted that subjects suspected of having or diagnosed as having Ebola and receiving therapeutically effective amounts of a VIV encoding an antibody that specifically targets Ebola virus, will display reduced severity or elimination of one or more symptoms associated with Ebola. It is further expected that administration of a VIV encoding an antibody that specifically targets Ebola virus in combination with one or more additional agents will have additive or synergistic effects.

These results will show that VIV encoding an antibody that specifically targets Ebola virus are useful in the treatment of Ebola or other infectious disease. Accordingly, a VIV encoding an antibody that specifically targets an infectious antigen is useful in methods comprising administering the VIV to a subject in need thereof for the treatment of an infectious disease.

### Example 2 - Use of VIV in the Prevention of Infectious Disease

This example demonstrates the use of the disclosed VIV, as demonstrated by a non-limiting example exemplified in Figure 1, in the prevention of Ebola virus or other infectious diseases. In this example, at least one of the "cargo" regions shown in Figure 1 encodes an antibody that specifically targets Ebola virus.

Subjects suspected of having an increased risk of contracting Ebola receive administrations of a prophylactically effective amount of a VIV encoding an antibody that specifically targets Ebola virus, either alone or in combination with one or more additional agents for the treatment or prevention of Ebola prior to entering an area in which risk of contracting Ebola is increased. VIV encoding an antibody that specifically targets Ebola virus and/or additional agents are administered orally, intranasally, intrathecally, intraocularly, intradermally, transmucosally, iontophoretically, topically, systemically, intravenously, subcutaneously, intraperitoneally, or intramuscularly according to methods known in the art or as described herein. Subjects will be evaluated daily for the presence and/or severity of signs and symptoms associated with Ebola, including, but not limited to, *e.g.,* fever, fatigue, malaise, weakness, reddened eyes, joint and muscle pain, headache, nausea, vomiting, hemorrhage, and death. Treatments are maintained until such a time as one or more signs or symptoms of Ebola are prevented.

It is rationally predicted that subjects suspected of having or diagnosed as having Ebola and receiving prophylactically effective amounts of a VIV encoding an antibody that specifically targets Ebola virus, will have a reduced risk of contracting Ebola. It is further expected that administration of VIV encoding an antibody that specifically targets Ebola virus in combination with one or more additional agents will have additive or synergistic effects in this regard.

These results will show that VIV encoding an antibody that specifically targets Ebola virus are useful in the prevention of Ebola or other infectious disease. Accordingly, a VIV encoding an antibody that specifically targets an infectious antigen is useful in methods comprising administering the VIV to a subject in need thereof for the prevention of an infectious disease.

### Example 3 - Use of VIV for Enhanced Wound Healing

This example demonstrates the use of the disclosed VIV, as demonstrated by a non-limiting example exemplified in Figure 1, for enhanced wound healing. In this example, at least one of the "cargo" regions shown in Figure 1 encodes platelet-derived growth factor (PDGF). Subjects with a wound (*e.g*., from accident, injury, or surgery) receive administrations of a therapeutically effective amount of a VIV encoding platelet-derived growth factor (PDGF), alone or in combination with one or more additional agents for treating or sterilizing a wound. VIV PDGF and/or additional agents are administered orally, intranasally, intrathecally, intraocularly, intradermally, transmucosally, iontophoretically, topically, systemically, intravenously, subcutaneously, intraperitoneally, or intramuscularly according to methods known in the art or as described herein. Subjects will be evaluated daily to determine the status of the wound. Treatments are maintained until such a time as the wound is healed and scarring is minimalized.

It is rationally predicted that subjects with a wound and receiving therapeutically effective amounts of a VIV PDGF will display enhanced wound healing. It is further expected that administration of VIV encoding PDGF in combination with one or more additional agents will have additive or synergistic effects.

These results will show that VIV encoding PDGF are useful for enhancing wound healing. Accordingly, a VIV encoding PDGF or a similar gene is useful in methods comprising administering the VIV to a subject in need thereof for the treatment of a wound.

### Example 4 - Use of VIV in the Treatment of Bone Injury

This example demonstrates the use of the disclosed VIV, as demonstrated by a non-limiting example exemplified in Figure 1, in the treatment of a bone injury. In this example, at least one of the "cargo" regions shown in Figure 1 encodes vascular endothelial growth factor (VEGF).

Subjects suspected of having or diagnosed as having a bone injury receive administrations of a therapeutically effective amount of a VIV encoding vascular endothelial growth factor (VEGF), alone or in combination with one or more additional agents for the treatment of the bone injury. VIV encoding VEGF and/or additional agents are administered orally, intranasally, intrathecally, intraocularly, intradermally, transmucosally, iontophoretically, topically, systemically, intravenously, subcutaneously, intraperitoneally, or intramuscularly according to methods known in the art or as described herein. Subjects will be evaluated weekly for the presence and/or severity of signs and symptoms associated with the bone injury to determine the rate and strength of healing. Treatments are maintained until such a time as the bone has healed.

It is rationally predicted that subjects suspected of having or diagnosed as having a bone injury and receiving therapeutically effective amounts of a VIV encoding VEGF will display reduced severity of injury and enhanced healing. It is further expected that administration of VIV encoding VEGF in combination with one or more additional agents will have additive or synergistic effects.

These results will show that VIV encoding VEGF are useful in the treatment of bone injuries or diseases. Accordingly, a VIV encoding VEGF or similar genes is useful in methods comprising administering a VIV to a subject in need thereof for the treatment of a bone injury or disease.

### Example 5 - Use of VIV in the Treatment of Hereditary Disease

This example demonstrates the use of the disclosed VIV, as demonstrated by a non-limiting example exemplified in Figure 1, in the treatment of cystic fibrosis (CF) or other hereditary genetic diseases. In this example, at least one of the "cargo" regions shown in Figure 1 encodes cystic fibrosis transmembrane conductance regulator (CFTR).

Subjects suspected of having or diagnosed as having (CF) receive administrations of a therapeutically effective amount of a VIV encoding cystic fibrosis transmembrane conductance regulator (CFTR), alone or in combination with one or more additional agents for the treatment of CF. VIV encoding CFTR and/or additional agents are administered orally, intranasally, intrathecally, intraocularly, intradermally, transmucosally, iontophoretically, topically, systemically, intravenously, subcutaneously, intraperitoneally, or intramuscularly according to methods known in the art or as described herein. Subjects will be evaluated weekly for the presence and/or severity of signs and symptoms associated with CF, including, but not limited to, *e.g.,* poor growth, persistent cough, thick sputum and mucus, wheezing, breathlessness, decreased ability to exercise, repeated lung infections, inflamed nasal passage, greasy stools, intestinal blockage, and poor weight gain. Treatments are maintained until such a time as one or more signs or symptoms of CF are ameliorated or eliminated.

It is rationally predicted that subjects suspected of having or diagnosed as having CF and receiving therapeutically effective amounts of a VIV encoding CFTR will display reduced severity or elimination of one or more symptoms associated with CF. It is further expected that administration of VIV encoding CFTR in combination with one or more additional agents will have additive or synergistic effects.

These results will show that VIV encoding CFTR are useful in the treatment of CF or other hereditary genetic diseases. Accordingly, a VIV encoding a mutated or otherwise defective gene related to a genetic disease is useful in methods comprising administering the VIV to a subject in need thereof for the treatment of the genetic disease.

### Example 6 - Use of VIV Containing E1 to Express Cargo

A vector according to Figure 2 was produced containing green fluorescent protein gene (GFP) as the cargo. DNA containing the complete Locus Control Region and E1 protein from human papillomavirus type 16 (NCBI accession number U89348) was chemically synthesized. Individual segments and/or coding sequences were initially synthesized. These were amplified by polymerase chain reaction (PCR) using synthetic oligonucleotide primers identical to the 5' end of green fluorescent protein gene and complementary to the 3' end of green fluorescent protein. The 5' primer was extended from its 5' end with the recognition site for BamHI or EcoRI endonucleases. The 3' primer was extended at its 3' end with the complement of BamHI, or EcoRI endonuclease recognition sites. The resulting amplified green fluorescent protein gene sequences were then digested with BamHI and EcoRI restriction endonucleases.

A lentiviral vector was obtained from System Biosciences, Inc. The plasmid was cleaved with BamHI and EcoRI enzymes, and mixed with excess amplified green fluorescent protein gene sequences in a 1:3 ratio of insert to vector.

Enzymatic activity was then stopped by heat inactivation at 70 degrees Celsius for 20 minutes. The above mixture was cooled to room temperature to allow annealing.

The annealing reactions were performed with bacteriophage T4 DNA ligase for 30 minutes at room temperature. 2.5 microliters of the resulting ligation mix were added to 25 microliters of STBL3 competent bacterial cells.

Transfection was then carried out by a brief (1 minute) heat-shock at 42 degrees Celsius.

Bacterial cells were streaked onto agar plates containing ampicillin to obtain bacterial cultures. These cultures were expanded in Luria broth.

To check for insertion of amplified green fluorescent protein gene sequences into the lentivirus vector packaging plasmid, DNA was extracted from the above bacterial cultures and purified by standard methods. Purified DNA was digested with the same endonucleases used to make the construct. Fragment lengths were analyzed by agarose gel electrophoresis, and the amplified green fluorescent protein gene sequences were verified by DNA sequencing using specific primers obtained from Eurofins MWG Operon LLC.

Lentivirus vector stocks were produced as follows. At least two lentiviral packaging plasmids plus the cargo plasmid were co-transfected into HEK cells where viral genes and genomic RNA are expressed, assembled into integrase-deficient lentivirus particles, and released into the culture medium. Cell-free supernatants were produced and collected during the interval of 3-10 days after transfection. Lentivirus particles were purified by standard procedures including a combination of methods that could include centrifugation, transient flow filtration, size exclusion chromatography, size exclusion filtration or ion exchange chromatography. The concentration and biological activity (transducing units per ml) for each stock were determined.

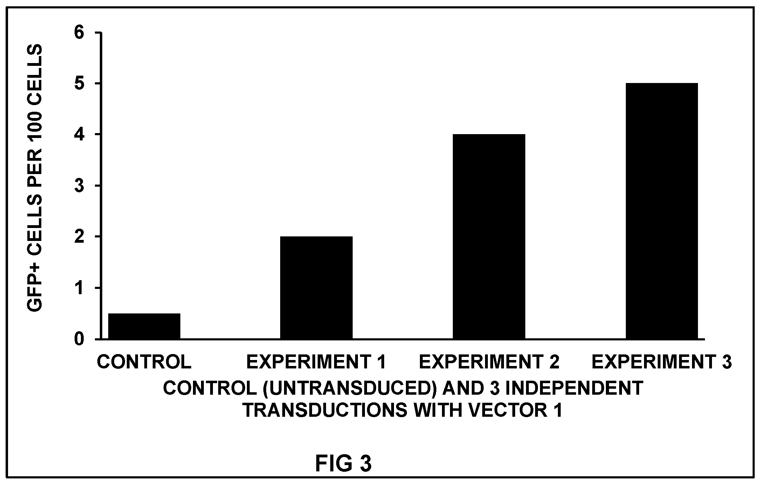

Mammalian cells, including 293 T cells, were used to test for lentivirus-derived episome formation, copy number and expression. The 293 T cells were transduced with integrase deficient lentivirus particles at a multiplicity of infection ranging from 1 to 10 in the presence of polybrene. Unabsorbed virus was removed by washing cells 3 hours after application, and cells were cultured for 3 days. Cells were observed in a fluorescence microscope and cells expressing GFP are counted. Untransduced 293 T cells were used as a negative control. Data was reported as GFP-positive cells per 100 viable cells in culture. A minimum of 300 cells were counted per microscope field and 5-10 fields are counted for each replicate experiment. Four independent transduction experiments comprising one negative control and three replicate experiments were performed to determine the frequency of transduced cells. The data is depicted in Figure 3, and shows expression of GFP across three replicate experiments.

### Example 7 - Use of VIV Containing E1 and E2 to Express Cargo

Vector 19, as shown in Figure 4, is constructed to contain both E1 and E2 initiator proteins. In this Example, genetic cargo is represented by a CMV/GFP expression cassette, and a suitable inducible promoter is also selected such that external addition of a compound that can induce the inducible promoter.

293 T cells are transduced with Vector 19 at a multiplicity of infection ranging between 1 and 20 transducing units per cell. 3 hours later, cells are washed with medium to remove unadsorbed virions and returned to culture. 12-24 hours after transduction, cells are treated with at least one dosage of a compound that can induce the inducible promoter. Upon addition of a compound that can induce the inducible promoter, E1 and E2 mRNA are transcribed from the episome and combine and assemble on the Locus Control Region Fragment 2 (LCR/F2) to trigger DNA replication. Lentivirus-derived episomes decay starting approximately 24-36 hours after the cessation of promoter induction. Protein products from the cargo in Vector 19 are measured by analytical flow cytometry.

## Claims

1. A viral delivery system comprising:
(a) a viral carrier, wherein the viral carrier has a defective integrase gene, wherein the viral carrier is a lentivirus, and wherein the defective integrase gene comprises mutations that inactivates the viral integrase gene;
(b) a heterologous viral episomal origin of replication;
(c) a sequence encoding at least one initiator protein specific for the heterologous viral episomal origin of replication, wherein expression of the sequence encoding the at least one initiator protein specific for the heterologous viral episomal origin of replication is under the control of a drug inducible promoter; and
(d) at least one gene, shRNA, siRNA, miRNA, or other gene-silencing RNA of interest.

2. The viral delivery system of claim 1, wherein the heterologous viral episomal origin of replication is from a papillomavirus, preferably wherein the heterologous viral episomal origin of replication is from bovine papillomavirus or human papillomavirus.

3. The viral delivery system of claim 1, wherein the at least one initiator protein specific for the heterologous viral episomal origin of replication is E1 or E2.

4. The viral delivery system of claim 1, wherein the system comprises two initiator proteins specific for the heterologous viral episomal origin of replication.

5. The viral delivery system of claim 4, wherein the two initiator proteins specific for the heterologous viral episomal origin of replication are E1 and E2.

6. The viral delivery system of claim 1, wherein the heterologous viral episomal origin of replication is from Epstein-Barr virus.

7. The viral delivery system of claim 6, wherein the initiator protein specific for the heterologous viral episomal origin of replication is EBNA-1.

8. A pharmaceutical composition comprising the viral delivery system of any one of claims 1 to 7 and at least one pharmaceutically acceptable carrier.

9. A viral delivery system according to any one of claims 1 to 7 for use in treating or preventing a disease.

10. The viral delivery system for use according to claim 9, wherein the disease is an infectious disease, preferably wherein the infectious disease is Ebola virus or Lassa fever virus, preferably wherein the gene, shRNA, siRNA, miRNA, or other gene-silencing RNA of interest is a gene which encodes an antibody specific for Ebola virus or Lassa fever virus.

11. The viral delivery system for use according to claim 9,wherein the disease is a genetic disease or disorder, preferably wherein the genetic disease is alcohol abuse, preferably wherein the gene, shRNA, siRNA, miRNA, or other gene-silencing RNA of interest is a gene which encodes brain-derived growth factor.

12. A viral delivery system according to any one of claims 1 to 7, for use in treating nerve damage, preferably wherein the gene, shRNA, siRNA, miRNA, or other gene-silencing RNA of interest is a gene which encodes nerve growth factor.

13. A viral delivery system according to any one of claims 1 to 7, for use in enhancing wound healing.

14. The viral delivery system for use according to claim 13, wherein the gene, shRNA, siRNA, miRNA, or other gene-silencing RNA of interest is a gene which encodes platelet-derived growth factor or vascular endothelial growth factor or wherein the wound is from an accident or injury, or the wound is from surgery, or the wound is a burn, or the wound is a bone nonunion.

## Patentansprüche

1. Virales Abgabesystem, das umfasst:
(a) einen viralen Träger, wobei der virale Träger ein defektes Integrase-Gen aufweist, wobei der virale Träger ein Lentivirus ist, und wobei das defekte Integrase-Gen Mutationen umfasst, die das virale Integrase-Gen inaktivieren;
(b) einen heterologen viralen episomalen Replikationsursprung;
(c) eine Sequenz, die für mindestens ein Initiatorprotein codiert, das für den heterologen viralen episomalen Replikationsursprung spezifisch ist, wobei die Expression der Sequenz, die für das mindestens eine Initiatorprotein codiert, das für den heterologen viralen episomalen Replikationsursprung spezifisch ist, unter der Kontrolle eines medikamenteninduzierbaren Promotors steht; und
(d) mindestens ein Gen, shRNA, siRNA, miRNA oder eine andere Gen-silencing-RNA von Interesse.

2. Virales Abgabesystem nach Anspruch 1, wobei der heterologe virale episomale Replikationsursprung von einem Papillomavirus stammt, vorzugsweise wobei der heterologe virale episomale Replikationsursprung von einem bovinen Papillomavirus oder einem humanen Papillomavirus stammt.

3. Virales Abgabesystem nach Anspruch 1, wobei das mindestens eine Initiatorprotein, das für den heterologen viralen episomalen Replikationsursprung spezifisch ist, E1 oder E2 ist.

4. Virales Abgabesystem nach Anspruch 1, wobei das System zwei Initiatorproteine umfasst, die für den heterologen viralen episomalen Replikationsursprung spezifisch sind.

5. Virales Abgabesystem nach Anspruch 4, wobei die beiden Initiatorproteine, die für den heterologen viralen episomalen Replikationsursprung spezifisch sind, E1 und E2 sind.

6. Virales Abgabesystem nach Anspruch 1, wobei der heterologe virale episomale Replikationsursprung vom Epstein-Barr-Virus stammt.

7. Virales Abgabesystem nach Anspruch 6, wobei das Initiatorprotein, das für den heterologen viralen episomalen Replikationsursprung spezifisch ist, EBNA-1 ist.

8. Pharmazeutische Zusammensetzung, die das virale Abgabesystem nach einem der Ansprüche 1 bis 7 und mindestens einen pharmazeutisch akzeptablen Träger umfasst.

9. Virales Abgabesystem nach einem der Ansprüche 1 bis 7 zur Verwendung bei der Behandlung oder Prävention einer Krankheit.

10. Virales Abgabesystem zur Verwendung nach Anspruch 9, wobei die Krankheit eine Infektionskrankheit ist, wobei vorzugsweise die Infektionskrankheit das Ebola-Virus oder das Lassa-Fieber-Virus ist, wobei vorzugsweise das Gen, die shRNA, die siRNA, die miRNA oder eine andere gen-silencing RNA von Interesse ein Gen ist, das einen für das Ebola-Virus oder Lassa-Fieber-Virus spezifischen Antikörper codiert.

11. Virales Abgabesystem zur Verwendung gemäß Anspruch 9, wobei die Krankheit eine genetische Krankheit oder Störung ist, wobei vorzugsweise die genetische Krankheit Alkoholmissbrauch ist, wobei vorzugsweise das Gen, die shRNA, die siRNA, die miRNA oder eine andere gen-silencing RNA von Interesse ein Gen ist, das für den vom Hirn abgeleiteten Wachstumsfaktor codiert.

12. Virales Abgabesystem nach einem der Ansprüche 1 bis 7, zur Verwendung bei der Behandlung von Nervenschäden, vorzugsweise, wobei das Gen, die shRNA, siRNA, miRNA oder eine andere gen-silencing RNA von Interesse ein Gen ist, das für den Nervenwachstumsfaktor codiert.

13. Virales Abgabesystem nach einem der Ansprüche 1 bis 7, zur Verwendung bei der Verbesserung der Wundheilung.

14. Virales Abgabesystem zur Verwendung nach Anspruch 13, wobei das Gen, die shRNA, die siRNA, die miRNA oder eine andere gen-silencing RNA von Interesse ein Gen ist, das für den Thrombozyten-Wachstumsfaktor oder den vaskulären endothelialen Wachstumsfaktor codiert, oder wobei die Wunde von einem Unfall oder einer Verletzung herrührt, oder die Wunde von einer Operation herrührt, oder die Wunde eine Verbrennung ist, oder die Wunde ein nicht-zusammengewachsener Knochen ist.

## Revendications

1. Système d'administration viral comprenant :
(a) un vecteur viral, le vecteur viral portant un gène d'intégrase défectueux, le vecteur viral étant un lentivirus, et le gène d'intégrase défectueux comprenant des mutations qui inactivent le gène d'intégrase viral,
(b) une origine de réplication épisomique virale hétérologue,
(c) une séquence codant pour au moins une protéine initiatrice spécifique de l'origine de réplication épisomique virale hétérologue, l'expression de la séquence codant pour l'au moins une protéine initiatrice spécifique de l'origine de réplication épisomique virale hétérologue étant régie par un promoteur inductible par un médicament, et
(d) au moins un gène, un ARNsh, un ARNsi, un ARNmi ou un autre ARN d'extinction génique d'intérêt.

2. Système d'administration viral selon la revendication 1, dans lequel l'origine de réplication épisomique virale hétérologue provient d'un papillomavirus, l'origine de réplication épisomique virale hétérologue provenant de préférence d'un papillomavirus bovin ou d'un papillomavirus humain.

3. Système d'administration viral selon la revendication 1, dans lequel l'au moins une protéine initiatrice spécifique de l'origine de réplication épisomique virale hétérologue est E1 ou E2.

4. Système d'administration viral selon la revendication 1, ledit système comprenant deux protéines initiatrices spécifiques de l'origine de réplication épisomique virale hétérologue.

5. Système d'administration viral selon la revendication 4, dans lequel les au moins deux protéines initiatrices spécifiques de l'origine de réplication épisomique virale hétérologue sont E1 et E2.

6. Système d'administration viral selon la revendication 1, dans lequel l'origine de réplication épisomique virale hétérologue provient du virus d'Epstein-Barr.

7. Système d'administration viral selon la revendication 6, dans lequel la protéine initiatrice spécifique de l'origine de réplication épisomique virale hétérologue est EBNA-1.

8. Composition pharmaceutique comprenant le système d'administration viral selon l'une des revendications 1 à 7 et au moins un vecteur pharmaceutiquement acceptable.

9. Système d'administration viral selon l'une quelconque des revendications 1 à 7, destiné à être utilisé dans le traitement ou la prévention d'une maladie.

10. Système d'administration viral destiné à être utilisé selon la revendication 9, dans lequel la maladie est une maladie infectieuse, la maladie infectieuse étant de préférence le virus Ebola ou le virus de la fièvre de Lassa, le gène, l'ARNsh, l'ARNsi, l'ARNmi ou autre ARN d'extinction génique d'intérêt étant de préférence un gène qui code pour un anticorps spécifique du virus Ebola ou du virus de la fièvre de Lassa.

11. Système d'administration viral destiné à être utilisé selon la revendication 9, dans lequel la maladie est une maladie ou un trouble génétique, la maladie génétique étant de préférence l'abus d'alcool, le gène, l'ARNsh, l'ARNsi, l'ARNmi ou autre ARN d'extinction génique d'intérêt étant de préférence un gène qui code pour un facteur de croissance dérivé du cerveau.

12. Système d'administration viral selon l'une quelconque des revendications 1 à 7, destiné à être utilisé dans le traitement des lésions nerveuses, le gène, l'ARNsh, l'ARNsi, l'ARNmi ou autre ARN d'extinction génique d'intérêt étant de préférence un gène qui code pour le facteur de croissance nerveuse.

13. Système d'administration viral selon l'une quelconque des revendications 1 à 7, destiné à être utilisé pour améliorer la cicatrisation des plaies.

14. Système d'administration viral destiné à être utilisé selon la revendication 13, dans lequel le gène, l'ARNsh, l'ARNsi, l'ARNmi ou autre ARN d'extinction génique d'intérêt est un gène qui code pour un facteur de croissance dérivé des plaquettes ou un facteur de croissance endothélial vasculaire, ou la plaie résultant d'un accident ou d'une blessure, ou la plaie résultant d'une intervention chirurgicale, ou la plaie étant une brûlure, ou la plaie étant une non-consolidation osseuse.
